# EUROPÄISCHE PATENTSCHRIFT

(11) **EP 3 219 361 B1**
(45) Veröffentlichungstag und Bekanntmachung des Hinweises auf die Patenterteilung: **17.10.2018**
(21) Anmeldenummer: 16202427.7
(22) Anmeldetag: 06.12.2016
(51) Int. Cl.: A61N 5/06

(54) **VORRICHTUNG ZUR SIMULTANEN SCHRÖPF- UND FARBLICHTTHERAPIE**
DEVICE FOR SIMULTANEOUS BLEED AND COLOURED LIGHT THERAPY
DISPOSITIF DE CHROMOTHÉRAPIE ET DE THÉRAPIE PAR VENTOUSES SIMULTANÉES

(30) Priorität: 16.03.2016 DE 102016104884
(43) Veröffentlichungstag der Anmeldung: 20.09.2017
(73) Patentinhaber: HeVaTech GmbH, 72661 Grafenberg (DE)
(72) Erfinder: Hengge, Tobias, 72661 Grafenberg (DE)
(74) Vertreter: Kohler Schmid Möbus Patentanwälte

(56) Entgegenhaltungen:
- US-A1- 2004 077 977
- US-A1- 2005 038 418
- US-A1- 2013 178 764
- US-A1- 2013 178 916

## Beschreibung

Die Erfindung betrifft eine Vorrichtung zur Ermöglichung einer simultanen Durchführung einer Schröpftherapie und einer Farblichttherapie, wobei die Vorrichtung sowohl eine Schröpftherapieanordnung mit einer Druckerzeugungseinheit zur Erzeugung von Vakuum in einem oder mehreren halboffenen Schröpfgefäßen, die mit ihren offenen Seiten zu behandelnde Körperoberflächen eines Patienten abdecken, einer Pumpe zur Vakuumerzeugung sowie einer Ventileinheit als auch eine Farblichttherapieanordnung mit einer Lichtquelle zur Erzeugung von Licht in einem Frequenzbereich innerhalb des sichtbaren Spektrums der elektromagnetischen Strahlung umfasst, mit dem die zu behandelnden Körperoberflächen des Patienten bestrahlt werden können.

Eine derartige Vorrichtung ist bekannt aus der DE 10 2007 018 863 B4.

Mit der Schröpftherapie lassen sich über Hautareale innere Organe beeinflussen. Sie wird angewandt als ausleitendes Verfahren zur Entlastung oder Anregung des Organismus. Darstellungen von Schröpfgläsern sind bereits aus dem alten Ägypten überliefert. Im klassischen Griechenland war das Schröpfen so geschätzt, dass die Schröpfglocke (neben dem Äskulap-Stab) zum Emblem des Arztes wurde.

In der Praxis ist die Schröpfkopfbehandlung der leichteste Zugang zum gestörten Gleichgewicht des Organismus. Wenn man eine gezielte Schröpftherapie an den Schröpforten durchführt, verschwinden oft mit einem Schlage viele spezielle Leiden. An den Wirbelsäulensegmenten entspringen Nervenfasern, die nicht nur zu einzelnen Organen ziehen, sondern auch zu bestimmten Hautarealen ("Headsche Zonen"). Über die Behandlung dieser Hautzonen, die in der Wirbelsäule denselben Ursprung haben, lassen sich auch rückgekoppelt Wirkungen auf die verknüpften Organe erzielen. Außerdem lassen sich die Wirkungen der Schröpfbehandlung auch mit den Funktionsmechanismen der Reflexzonen oder der Akupunkturpunkte am Rücken erklären.

Auslösende Situationen für den Aufbau einer Schröpfzone liegen in der Regel vor, wenn durch äußere oder innere Faktoren ein Organ in der Tiefe gestört ist (Organirritationszone), wenn ein Gelenk blockiert ist (Gelenkirritationszone), wenn ein Fokus ein Segment oder Funktionskreis imitiert (Herdreflexzone) oder wenn ein psychischer Faktor zu einer derartigen Irritation führt (psychosomatische Beschwerden).

Während die Schröpfkopfbehandlung in früheren Jahrhunderten nur mittels eines statischen Vakuums über einer begrenzten Hautzone erfolgte, welches auf unterschiedliche Weisen erzeugt und für einen bestimmten Zeitraum aufrecht erhalten wurde, kommen in neuerer Zeit auch Vakuum-Massagegeräte zur Anwendung, bei denen über der Schröpfzone ein pulsierendes Vakuum oder in einem intermittierenden Wechsel Unterdruck und Überdruck aufgebaut werden.

Ein solches Vakuum-Massagegerät mit veränderbarer Saugfrequenz ist z.B. in der DE-U 202 03 037 beschrieben. Danach soll mit Hilfe einer Kolbenpumpe periodisch jeweils ein Unterdruck- und darauf folgend ein Überdruckpuls erzeugt und an die Schröpfglocke weitergeleitet werden, so dass eine stehende Welle mit einem in einer gewünschten Frequenz oszillierenden Vakuum über der Schröpfzone entsteht, wobei die Oszillationsfrequenz mittels eines elektronischen Frequenzmodulators individuell eingestellt werden soll.

Ein ähnliches pneumatisches Massagegerät beschreibt die DE 102 11 411 C1, in welcher zusätzlich eine elektronische Steuereinheit vorgeschlagen wird, mit deren Hilfe zwei elektromagnetisch betätigbare Steuerventile so angesteuert werden sollen, dass wahlweise entweder ein pulsierender Unterdruck oder ein pulsierender Überdruck am Behandlungsgerät erzeugt wird. Dies soll dadurch bewirkt werden, dass beide Steuerventile in einer Verbindungsleitung zwischen dem Behandlungsgerät und einer elektromotorisch angetriebenen Kolbenpumpe angeordnet sind, wobei das eine Steuerventil den Arbeitsraum der Kolbenpumpe mit dem Behandlungsgerät und das andere Steuerventil die Verbindungsleitung mit der Umgebungsatmosphäre verbinden oder dagegen absperren kann.

Eine weitere Verbesserung derartiger Vakuum-Massagegeräte ist aus der DE 20 2004 370 U1 bekannt. Damit soll nicht nur die Oszillationsfrequenz des Vakuums, sondern auch dessen Amplitude mittels eines Vakuumreglers stufenlos eingestellt werden, um eine sanft geregelte, pulsierende Schröpfkopfmassage zu erreichen.

Alle diese bekannten Schröpftherapiegeräte ermöglichen jedoch immer nur in einem sehr begrenzten Regelbereich eine für eine wirkungsvolle Therapie ausreichend hohe Leistungsaufnahme. Die meisten dieser Geräte haben eine zu geringe Dynamik um tiefer liegende Muskel- und Gewebeschichten zu erreichen. Daher eignen sich diese Geräte zwar sehr gut zur Oberflächenmassage, jedoch nicht zur Lösung von Ablagerungen und Blockaden in tieferen Gewebeschichten des Patienten. Insbesondere können damit Ablagerungen in Muskeln, Gelenke und Knorpel ohne invasiven Eingriff und auch bei geringer Leistungsaufnahme nicht wirkungsvoll aufgelöst werden.

Einen erheblich weiteren Regelbereich, eine höhere Leistungsaufnahme und Dynamik der erzeugten Pulsationen und damit eine verbesserte Möglichkeit zur individuellen Anpassung der wesentlichen Parameter der Schröpftherapie an die Bedürfnisse des jeweiligen Patienten erreicht eine Vorrichtung und das zugehörige Betriebsverfahren, wie sie etwa in der DE 10 2007 040 053 B3 beschrieben sind, indem eine zweite Druckerzeugungseinheit vorgesehen ist, die mindestens ein Druckspeichergefäß zur Speicherung eines Über- oder Unterdruckes umfasst, welches über eine zweite Ventileinheit mit einem oder mehreren der Schröpfgefäße verbunden werden kann, sowie einen zweiten elektronischen Regler zur Ansteuerung der zweiten Ventileinheit.

Dieser pulsierenden Vakuumtherapie bescheinigen verschiedene Studien klinisch relevante Verbesserungen bei Arthrose, Chronische Schmerzen, Lendenwirbelsyndrom.

Daneben gibt es auch es mehrere Studien zur physiologischen Wirksamkeit einer Farblichttherapie, insbesondere mit Licht der Wellenlängen 410, 420, 453nm. Dieses Licht führt nämlich unter anderem zur Freisetzung von Stickstoffmonoxid (NO) in der Haut, dies führt zu einer systematischen Steigerung der Blutflussrate und Absenkung des Blutdrucks.

Auch im Bereich der Schutzrechte wird die Farblichttherapie häufig erwähnt, weiterentwickelt und verbessert. So beschreibt zum Beispiel die DE 295 10 124 U1 ein Gerät für Farblicht-Therapie, die DE 20 2004 004 476 U1 eine Brille mit Wechselfunktion für UV-Schutz und Farblichttherapie oder die EP 1 829 581 B1 eine Brause mit Einrichtung zur Farblichttherapie.

Damit wird jedoch jeweils entweder "nur" die Schröpftherapie als solche oder "nur" die Farblichttherapie verbessert beziehungsweise optimiert. Die eingangs zitierte DE 10 2007 018 863 B4 hingegen beschreibt bereits eine Vorrichtung für die gleichzeitige Vakuummassage und Farbbestrahlung, also eine Kombinationstherapie aus Schröpfen und Farblichtanwendung. Sowohl der Farblichttherapie als auch der Schröpftherapie wird in zahlreichen wissenschaftlichen Studien hohe klinische Relevanz in der Schmerztherapie bescheinigt. Durch die Kombination der beiden Therapieformen kann mithin eine noch höhere Wirksamkeit erreicht werden.

Allerdings bleiben trotzdem immer noch zahlreiche Nachteile dieses des Stands der Technik:
Wegen der angewendeten passiven Filterung und der vorgeschlagenen dezentralen Anordnung ergibt sich leider eine erhebliche Ineffizienz des bekannten Kombinationsverfahrens. Es kommt zu erheblichen Verlusten auf der Strecke zwischen der zu behandelnden Körperstelle und der Lichtquelle. Ferner führt ein breites Frequenzspektrum der Lichtquelle mit der passiven Filterung zu beträchtlichen Intensitätsverlusten beim therapeutisch nutzbaren Farblicht. Als weiterer Nachteil ist festzustellen, dass eine exakte Erzeugung der Wellenlänge, die für die Ausschüttung von NO wesentlich ist (siehe wissenschaftliche Studien), durch passive Filterung kaum möglich ist. Weiterhin ist die Starrheit der Anordnung durch relativ sperrige, vor allem aber nicht-elastische Applikatoren ziemlich nachteilig. Dadurch wird eine gleichmäßige Bestrahlung der zu behandelnden Körperstelle praktisch unmöglich gemacht. Weiter ist das Anbringen und Fixieren dieser starren Applikatoren an einigen Körperstellen zumindest für die Dauer einer normalen Behandlung oftmals gar nicht praktikabel.

Darüber hinaus wird auf das Dokument US2004/0077977 A1 verwiesen.

Aufgabe der vorliegenden Erfindung ist es demgegenüber, eine gattungsgemäße Vorrichtung der eingangs beschriebenen Art mit möglichst einfachen technischen Mitteln dahin gehend zu verbessern, dass unaufwändig und kostengünstig eine effektive, flexible, gleichmäßige Anwendung auf weitestgehend jeder beliebigen Körperstelle bequem ermöglicht wird. Außerdem soll die erforderliche Wellenlänge möglichst genau und mit maximalem Wirkungsgrad auf die zu behandelnde Körperstelle eingestrahlt werden. Weiter sollen auch noch die Vorteile der pulsierenden Schröpftherapie mit geregeltem Vakuum, wie sie in DE 10 2007 040 053 B3 beschrieben sind, die Wirksamkeit der Gesamt-Therapie nochmals wesentlich erhöhen.

Erfindungsgemäß wird diese -genau besehen relativ komplexe-Aufgabe auf ebenso überraschend einfache wie wirkungsvolle Art und Weise mit ohne Weiteres zur Verfügung stehenden oder zumindest leicht zu beschaffenden technischen Mitteln dadurch gelöst, dass die Druckerzeugungseinheit der Schröpftherapie-anordnung zur Erzeugung von Vakuumimpulsen mit variabel einstellbarer Amplitude ausgestaltet ist, wobei die Ventileinheit eine stufenlose Variation der erzeugten Vakuumamplitude ermöglicht, und wobei ein elektronischer Regler zur Regelung der erzeugten Vakuumamplitude durch Ansteuerung der Druckerzeugungseinheit und/oder der Ventileinheit vorhanden ist, dass die halboffenen Schröpfgefäße aus flexiblem Material aufgebaut und auf einer Vorderseite einer flexiblen Kunststoffmatte angeordnet sind, wobei die Schröpfgefäße jeweils über eine Durchgangsöffnung mit der gegenüberliegenden Rückseite der flexiblen Kunststoffmatte verbunden sind, dass eine Deckeleinheit vorgesehen ist, die im Betriebszustand zusammen mit der Rückseite der flexiblen Kunststoffmatte einen Hohlraum einschließt, in welchen eine Druckluftleitung mündet, die mit der Druckerzeugungseinheit verbunden ist, dass die Lichtquelle der Farblichttherapieanordnung in dem von der flexiblen Kunststoffmatte und der Deckeleinheit eingeschlossenen Hohlraum angeordnet ist, und dass eine elektronische Steuereinrichtung vorhanden ist, mit der die Schröpftherapieanordnung und die Farblichttherapieanordnung derart angesteuert werden können, dass sie entweder in Kombination miteinander simultan oder auch jeweils einzeln für sich betreibbar sind.

Dadurch werden auf simple Weise die Vorteile des oben beschriebenen bekannten kombinierten Schröpf- und Farblichttherapiegerätes gemäß der gattungsbildenden DE 10 2007 018 863 B4 genutzt, wobei aber die genannten Nachteile vermieden werden und zudem auch noch die günstigen Eigenschaften der pulsierenden Schröpftherapie mit geregeltem Vakuum gemäß DE 10 2007 040 053 B3 nutzbar gemacht werden können.

Durch die erfindungsgemäß eingesetzten Vakuumimpulse mit variabel einstellbarer Amplitude kann das Gewebe des Patienten an der zu behandelnden Stelle lokal gedehnt, gelockert bzw. vorgespannt werden und es kommt zu vermehrter Durchblutung, wobei etwaige Gefäßverletzungen durch zu hohe Druckgradienten vermieden werden. Damit kann eine überlagerte Vakuumwelle in einem Frequenzbereich bis etwa 10Hz direkt auf die zu behandelnde Stelle wirken. Durch dieses pulsierende Schröpfen wird das Gewebe lokal gedehnt und sanft massiert, es wird mit Nährstoffen angereichert (Blut, Lymphe, etc.).

Wird nun dieser massagebehandelten Körperregion im Rahmen der Farblichttherapie Licht in bestimmter Wellenlänge zugeführt, kommt es im Gewebe der Haut zur Produktion von Stickstoffmonoxid (NO) in unbedenklicher Menge. Einer der wichtigsten durch NO vermittelten Effekte ist die Vasodilatation (Wachstum und Neubildung von Blutgefäßen) aufgrund der Entspannung der glatten Muskulatur. Einer der wichtigsten durch NO vermittelten Effekte ist die Vasodilatation (Wachstum und Neubildung von Blutgefäßen) durch Entspannung der glatten Muskulatur. Dies führt dazu, dass die intrazelluläre Kalziumkonzentration abnimmt, somit wird der Muskel entspannt und neue Blutgefäße werden gebildet.

Durch die Vereinheitlichung beider Therapien in einer erfindungsgemäß elastischen Anordnung kann diese neue Kombinations-Therapieform sehr flexibel auf der Haut angewandt werden. Die Wirksamkeit wird durch die Kombination wesentlich erhöht. Somit unterstützen die beiden Therapien sich gegenseitig in der Schmerztherapie.

Je nach Vakuumamplitude und eingestellter Pulsationsfrequenz können auch tiefer liegende Ablagerungen gelöst und durch das Vorspannen des Gewebes effektiv abgebaut werden. Die Tiefenwirkung kann der Therapeut über einen Unterdruckregler bestimmen.

Die elektronische Regelung ermöglicht, bei geringer Leistungsaufnahme, eine hohe Dynamik. Die gewünschte Einstellung kann der Therapeut sehr leicht durch weiter unten beschriebene Stellglieder für Vakuum, Frequenz und Amplitude wählen. Der statische Unterdruck wird durch eine elektronisch geregelte Vakuumpumpe und ein einfaches Stellventil geregelt. Schnelle Druckänderungen werden durch einen Druck- bzw. Vakuumspeicher mit separater Pumpe über die beiden elektronisch angesteuerten Stellventile dynamisch aufgeschaltet. Die Elektronik verhindert, dass die Regelglieder gegeneinander arbeiten. Dies führt zu einer optimalen Leistungsanpassung des Systems.

Besonders einfach aufgebaut ist die erfindungsgemäße Vorrichtung bei Varianten, in welchen die halboffenen Schröpfgefäße sowie die flexible Kunststoffmatte, vorzugsweise auch die Deckeleinheit, aus demselben flexiblen Material, insbesondere aus thermoplastischen Elastomeren, Silikon oder Polypropylen aufgebaut sind. Dies ermöglicht ein einfaches Anbringen der Vorrichtung auf den zu behandelnden Körperstellen durch die flexible Verformbarkeit der Matte, eine gute Hautverträglichkeit der Materialien, einfache Reinigung und Handhabung.

Bei weiteren vorteilhaften Ausführungsformen der erfindungsgemäßen Vorrichtung sind die halboffenen Schröpfgefäße einstückig mit der flexiblen Kunststoffmatte ausgeführt. Auf diese Weise sind nur sehr wenige Einzelteile erforderlich. Im Betrieb ist die Verschmutzung und Verkeimung vor allem in den Anschlüssen und Zwischenräumen im Gegensatz zu einzeln angebrachten Schröpfgefäßen äußerst gering.

Bevorzugt sind auch Ausführungsformen, bei denen die Druckerzeugungseinheit Vakuumimpulse mit variabel einstellbarer Frequenz erzeugen kann. Dies ermöglicht eine individuelle Einstellung, je nach Therapie und Anwendung, von sanften Massageimpulsen bis zu tief wirkender Gelenksbehandlung (siehe auch Studie zu Gonarthrose von HeVaTech).

Bei einer Klasse von vorteilhaften Ausführungsformen der Erfindung ist die flexible Kunststoffmatte aus einem transparenten oder opaken Material aufgebaut, wobei die dem Hohlraum zugewandte Seite der Deckeleinheit vorzugsweise verspiegelt ist. Dadurch wird das Therapie-Licht exakt an die Körperstellen gebracht, wo es gewünscht ist und benötigt wird. Dies ist ein wesentlicher Vorteil gegenüber dem Stand der Technik, da Marktübliche Geräte relativ viel Licht nutzlos in die Umgebung emittieren.

In einer alternativen Klasse von Ausführungsformen der erfindungsgemäßen Vorrichtung kann aber auch die Lichtquelle der Farblichttherapieanordnung eine Vielzahl von Beleuchtungselementen, insbesondere Lichtleiter und/oder farbige LEDs und/oder OLEDs, umfassen, welche auf der Vorderseite oder mit optischer Verbindung zur Vorderseite der flexiblen Kunststoffmatte geometrisch auf Lücke zu den Schröpfgefäßen angeordnet sind. Auch dadurch wird eine flächige Kombinations-Therapie bei möglichst geringem Energiebedarf ermöglicht, weil wiederum das Therapie-Licht genau an diejenige Stelle gelenkt wird, wo es gewünscht ist.

Eine weitere besonders bevorzugte Klasse von Ausführungsformen zeichnet sich dadurch aus, dass sämtliche im Hohlraum zwischen der flexiblen Kunststoffmatte und der Deckeleinheit angeordneten optischen und elektronischen Bauteile jeweils fluiddicht und vorzugsweise auch elastisch gekapselt sind. Damit wird eine einfache Reinigung sowie eine unproblematische Desinfektion der kompletten Einheit ermöglicht. Außerdem wird ein Eindringen von Flüssigkeiten in die Elektronik zuverlässig verhindert. Von Anwenderseite ist trotzdem keine besondere Handhabung erforderlich.

Bevorzugt sind Weiterbildungen dieser Klasse von Ausführungsformen, bei denen eine Desinfektionsbadewanne vorgesehen ist, in welcher die Baueinheit aus flexibler Kunststoffmatte und Deckeleinheit einschließlich in deren Hohlraum angeordneter Bauteile mittels Desinfektionsflüssigkeit desinfiziert werden kann, und dass in die Desinfektionsbadewanne eine elektrische Ladestation zur Aufladung von elektrisch aufladbaren Bauteilen im Hohlraum integriert ist. Alle elektronischen Bauteile sind hierbei in geschützten Bereichen untergebracht. Es ist keine elektrische Verbindung zwischen Schröpfmatte und elektrischer Ladestation erforderlich. Das Laden und Desinfizieren kann in einem einzigen Arbeitsgang erfolgen, wodurch ein erheblicher Schutz für den Patienten und Anwender bezüglich der Hygiene und elektrischen Gefährdungen erreicht wird.

Besonders vorteilhaft sind auch Ausführungsformen der erfindungsgemäßen Vorrichtung, bei welchen die Lichtquelle der Farblichttherapieanordnung farbiges Licht von wählbarer, unterschiedlicher Wellenlänge und wählbarer, variabler Intensität erzeugen kann, wobei eine Regelvorrichtung vorgesehen ist, mittels derer sowohl die Energie als auch die Amplitude des von der Lichtquelle erzeugten Farblichts geregelt werden kann. Die Sollvorgabe für die Lichteinheit kann entweder über in der Rückseite der Matte integrierte Tasten oder über eine Funkschnittstelle erfolgen. Durch die aktive Steuer/Regeleinheit wird -im Gegensatz zum Stand der Technik, wo eine Steuerung durch Farbfilter vorgeschlagen wird- eine hohe Effizienz und Genauigkeit der Wellenlänge erreicht. Die präzise Anwendung bestimmter Wellenlängen hat bei der Farblichttherapie eine besondere Bedeutung, da zum Beispiel die oben beschriebene förderliche NO-Bildung nur bei ganz speziellen Wellenlängen erfolgt. Dies ermöglicht außerdem sehr flexible Einsätze, insbesondere auch für zukünftige Anwendungen der Erfindung, die noch klinisch untersucht werden müssen.

Geometrisch ganz besonders einfach aufgebaut sind Weiterbildungen dieser Ausführungsformen, bei denen die Regelvorrichtung für das von der Lichtquelle erzeugte Farblicht in dem von der flexiblen Kunststoffmatte und der Deckeleinheit umschlossenen Hohlraum angeordnet ist. Auf diese Weise ist keine externe Lichtquelle erforderlich. Dadurch verringern sich die Intensitätsverluste auf der Strecke zwischen Lichtquelle und zu behandelnder Hautregion erheblich, was eine besonders hohe Energieeffizienz und damit eine maximale Wirksamkeit zur Folge hat.

Eine weitere vorteilhafte Ausführungsform der Erfindung ist dadurch gekennzeichnet, dass in dem von der flexiblen Kunststoffmatte und der Deckeleinheit umschlossenen Hohlraum eine autarke Energiequelle zur Versorgung der Lichtquelle angeordnet ist, wobei die Energiequelle vorzugsweise Batterien und/oder wiederaufladbare Akkumulatoren und/oder Supercap-Kondensatoren enthält. Dies ermöglicht eine sehr einfache Handhabung. Es sind keine externen Energiequellen bei der Behandlung erforderlich. Ebenso entfällt eine komplizierte Handhabung von elektrischen Anschlüssen und die Vorrichtung entfaltet im Betrieb nur ein äußerst geringes elektrisches Gefährdungspotential.

Bevorzugt sind auch Ausführungsformen der erfindungsgemäßen Vorrichtung, bei denen zumindest die in dem von der flexiblen Kunststoffmatte und der Deckeleinheit umschlossenen Hohlraum angeordneten Teile der Farblichttherapieanordnung mechanisch flexibel gestaltet sind, was eine flexible Anbringung an den zu behandelnden Körperstellen erleichtert oder überhaupt erst ermöglicht.

Bei einer Klasse von Ausführungsformen der Erfindung umfasst die Lichtquelle der Farblichttherapieanordnung eine Vielzahl von quasi-punktförmigen Farblichterzeugenden Elementen, vorzugsweise LEDs und/oder OLEDs und macht so eine optimale Lichtverteilung auf die zu behandelnden Körperstellen möglich.

Bei einer Klasse von alternativen Ausführungsformen hingegen ist die Lichtquelle der Farblichttherapieanordnung flächig ausgedehnt gestaltet. Auch damit lässt sich eine optimale Lichtverteilung auf die zu behandelnden Körperstellen erzielen, vor allem bei der Realisierung in einem elastischen Schröpfkopf zur Behandlung bei Arthrosen (siehe hierzu auch die Studie zur Behandlung von Gonarthrose mit einem elastischen Gelenkschröpfkopf).

Die halboffenen Schröpfgefäße der erfindungsgemäßen Vorrichtung werden -wie oben beschrieben- bei der Behandlung eines Patienten auf ausgewählte Hautzonen aufgesetzt und sodann zur Durchführung der gewünschten Massage mit einem pulsierenden Unterdruck beaufschlagt. In jedem Fall entsteht dabei immer ein von der Haut des Patienten weg in Richtung auf die erste Druckerzeugungseinheit gerichteter Druckgradient in den Zuleitungen zu den Schröpfgefäßen. Dieser Druckgradient bewirkt stets eine Massenströmung von Luft und anderen Partikeln von den entsprechenden Hautzonen in das Innere des Schröpftherapiegeräts. In diesem Strom werden in der Regel auch Schmutz- und Fluidpartikel von der Haut des Patienten enthalten sein, die von Hautverunreinigungen, Schweiß etc. herrühren. Insbesondere kann diese Massenströmung auch Keime und Krankheitserreger enthalten. Daher ist es erforderlich, diejenigen Teile der Apparatur, die mit dieser Massenströmung in Berührung kommen, regelmäßig zu reinigen, vorzugsweise nach jeder einzelnen Behandlung eines Patienten.

Um die Apparatur ohne großen Zusatzaufwand möglichst lange in einem sauberen, hygienischen und damit betriebsbereiten Zustand zu halten, ist bei ganz besonders bevorzugten Ausführungsformen der Erfindung vorgesehen, dass mindestens einem, vorzugsweise allen Schröpfgefäßen ein Fluidabsorber und/oder ein Feststofffilter nachgeschaltet ist, welcher in das oder die Schröpfgefäße eindringende Fluide und/oder Feststoffe von der Druckerzeugungseinheit abhält. Der Fluidabsorber und/oder der Feststofffilter fängt die unerwünschten Partikel aus der oben beschriebenen Massenströmung ab, von wo die Partikel dann einfach durch Reinigung des Absorber bzw. Filters aus dem erfindungsgemäßen Schröpftherapiegerät entfernt werden können.

Meist münden bei simultaner Anwendung mehrerer Schröpfgefäße die von diesen in Richtung auf die Druckerzeugungseinheit abgehenden Zuleitungen in einer gemeinsamen Sammelleitung. Zur Einsparung von allzu vielen Teilen, die einerseits Kosten verursachen und Platz beanspruchen, andererseits auch regelmäßig gereinigt, gewartet und gegebenenfalls ausgetauscht werden müssen, ist es vorteilhaft, wenn lediglich ein einziger Fluidabsorber und/oder ein einziger Feststofffilter an einer geeigneten, möglichst leicht zugänglichen Stelle in der Sammelleitung eingebaut wird. Von da kann der Absorber bzw. der Filter beispielsweise vor und/oder nach jeder Behandlung eines Patienten entnommen, gereinigt und wieder in die Sammelleitung eingesetzt werden.

Zur Feststellung der tatsächlichen Druckverhältnisse ist bei besonders bevorzugten Ausführungsformen des erfindungsgemäßen Schröpftherapiegerätes an mindestens einem, vorzugsweise an allen Schröpfgefäßen ein Drucksensor zur Ist-Wert-Bestimmung des in den Schröpfgefäßen aktuell herrschenden Unterdruckes vorgesehen.

Der Drucksensor kann in das jeweilige Schröpfgefäß integriert sein und direkt in den Vakuumraum des halboffenen Schröpfgefäßes ragen, was natürlich die genauesten Druckwerte liefert. Falls dies aus Platzgründen nicht möglich ist, kann der Drucksensor aber auch in der vom Schröpfgefäß in Richtung auf die Druckerzeugungseinheit abgehenden Druckleitung, möglichst in der Nähe des entsprechenden Schröpfgefäßes angeordnet sein. Als kostengünstige Lösung bei simultaner Verwendung einer Vielzahl von Schröpfgefäßen kommt auch die Anordnung eines einzigen zentralen Drucksensors in der oben beschriebenen Sammelleitung in Frage. Hier wird dann allerdings nur ein mittlerer Druckverlauf gemessen, der jedoch durchaus für die tatsächlichen Druckverläufe an den einzelnen Schröpfgefäßen ausreichend repräsentativ sein kann.

Von diesen Ausführungsformen kann mit besonderem Vorteil in Weiterbildungen Gebrauch gemacht werden, bei denen eine Steuereinrichtung vorgesehen ist, die den vom Drucksensor gemessenen IstWerts mit einem eingebbaren Soll-Wert vergleicht und den ersten und den zweiten elektronischen Regler entsprechend ansteuert.

Eine wichtige Variante dieser Weiterbildungen zeichnet sich dadurch aus, dass ein Stellglied -beispielsweise in Form eines Dreh- oder Schiebe-Potentiometers- vorgesehen ist, mit dem ein Soll-Wert für einen minimalen Unterdruck (="Grundvakuum") an den Schröpfgefäßen in die Steuereinrichtung eingegeben werden kann.

Alternativ oder ergänzend kann bei weiteren Varianten des erfindungsgemäßen Schröpftherapiegerätes ein Stellglied zur Eingabe eines Soll-Wertes für die maximale Amplitude des pulsierenden Vakuums an den Schröpfgefäßen und/oder ein Stellglied zur Eingabe eines Soll-Wertes für die Frequenz des pulsierenden Vakuums an den Schröpfgefäßen in die Steuereinrichtung vorgesehen sein. Vorteilhafterweise werden alle drei genannten Varianten gleichzeitig verwirklicht, um eine möglichst flexible und präzise Anpassung der wesentlichen Betriebsparameter der Apparatur an die individuellen Erfordernisse bei der Behandlung eines Patienten sicher zu stellen. Die entsprechenden Stellglieder können so ausgelegt sein, dass sie eine rasterbare Einstellung diskreter Parameterwerte oder aber eine kontinuierliche, stufenlose Einstellung derselben ermöglichen.

Vorteilhaft zum Betrieb einer erfindungsgemäß Kombinationsvorrichtung mit Schröpf- und Farblichttherapiegerät der oben beschriebenen Art ist ein Verfahren, bei weichem ein zeitlich nicht oder nur langsam veränderliches Grundvakuum in den Schröpfgefäßen erzeugt wird, wobei dem Grundvakuum schnelle Über- und/oder Unterdruckpulse überlagert werden, um auf der Haut des Patienten im Bereich der Behandlungszone(n) eine "stehende Welle" von pulsierendem Vakuum entstehen zu lassen.

In der Praxis haben sich Verfahrensvarianten als besonders günstig erwiesen, bei denen sich das Grundvakuum in einem Frequenzbereich f1 < 1Hz, vorzugsweise f1 < 0,1 Hz verändert, und die schnellen Druckpulse mit Frequenzen f2 ≥ 0,5Hz, vorzugsweise 1Hz ≤ f2 ≤ 10Hz variieren.

Besonders bevorzugt sind Verfahrensvarianten, bei denen mittels der oben beschriebenen Stellglieder gewünschte Soll-Werte für das Grundvakuum und für die Amplitude des pulsierenden Vakuums eingegeben werden und im Betrieb laufend ein Vergleich mit dem vom Drucksensor gemessenen Ist-Wert vorgenommen wird, wobei dann die Steuereinrichtung den ersten und den zweiten elektronischen Regler entsprechend so ansteuert, dass die Differenz zwischen Soll-Wert und Ist-Wert minimiert bzw. auf einen maximal zulässigen Wert begrenzt wird.

Vorteilhaft ist auch eine Verfahrensvariante, bei der mittels des entsprechenden Stellgliedes ein gewünschter Soll-Wert für die Frequenz f2 eingegeben wird, ein Vergleich mit dem vom Drucksensor gemessenen Ist-Wert vorgenommen wird, und mittels der Steuereinrichtung die ersten und den zweiten elektronischen Regler entsprechend so angesteuert werden, dass die Differenz zwischen Soll-Wert und Ist-Wert minimiert bzw. auf einen maximal zulässigen Wert begrenzt wird.

Zur Erhöhung der Genauigkeit einer individuellen Einstellung der relevanten Apparatur-Parameter auf die jeweiligen Bedürfnisse eines zu behandelnden Patienten sind Verfahrensvarianten besonders günstig, bei denen eine stufenlose Regelung der Amplitude des Grundvakuums und/oder der Frequenz f2 und/oder der Amplitude der schnellen Druckpulse erfolgt.

Weitere Merkmale und Vorteile der Erfindung ergeben sich aus der nachfolgenden detaillierten Beschreibung eines Ausführungsbeispiels der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein. In der schematischen Zeichnung ist ein Ausführungsbeispiel der Erfindung dargestellt, welches in der nachfolgenden Beschreibung näher erläutert wird.

Es zeigen:
- Fig. 1: einen schematischen Schnitt durch eine Ausführungsform des erfindungsgemäßen Kombinationsgeräts für pulsierende Schröpftherapie sowie simultan für Farblichttherapie auf derselben zu behandelnden Hautregion des Patienten; und
- Fig. 2: einen schematisierten Schnitt durch eine Desinfektionswanne mit berührungsloser Ladestation für die elektrischen und elektronischen Komponenten der erfindungsgemäßen Vorrichtung.

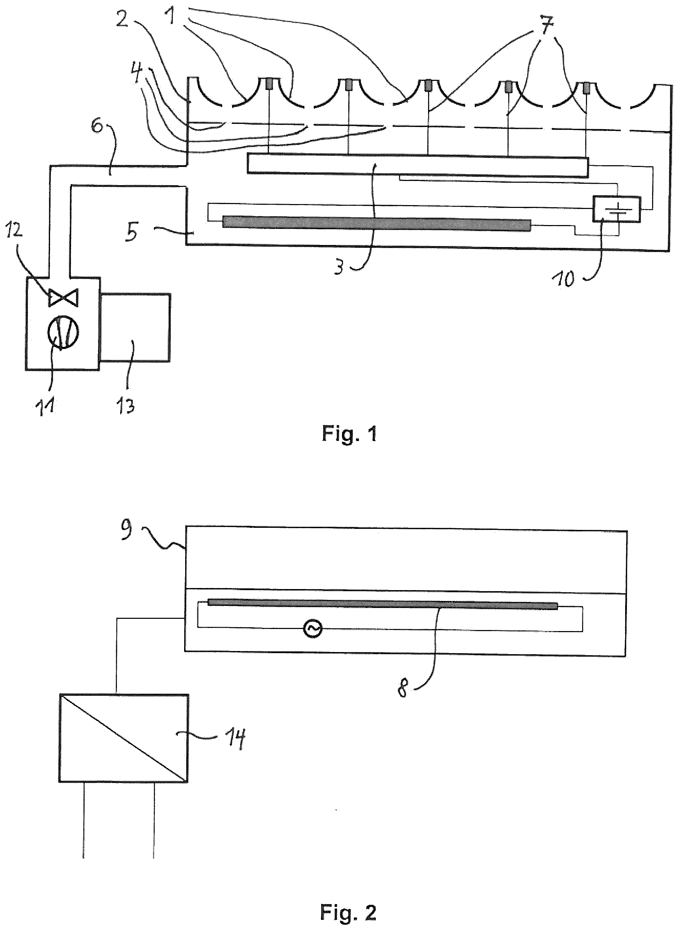

Die in Fig. 1 stark schematisiert dargestellte Ausführungsform eines erfindungsgemäßen Kombinationstherapiegerätes ermöglicht eine simultane, aber auch eine zeitlich und räumlich getrennte Durchführung einer Schröpftherapie und einer Farblichttherapie. Die Vorrichtung umfasst sowohl eine Schröpftherapieanordnung mit einer Druckerzeugungseinheit zur Erzeugung von Vakuum in einem oder mehreren halboffenen **Schröpfgefäßen 1,** die mit ihren offenen Seiten zu behandelnde Körperoberflächen eines Patienten abdecken, einer **Pumpe 11** zur Vakuumerzeugung sowie einer **Ventileinheit 12** als auch eine Farblichttherapieanordnung mit einer **Lichtquelle 3** (meist mit integrierter Steuer-/Regeleinheit) zur Erzeugung von Licht in einem Frequenzbereich innerhalb des sichtbaren Spektrums der elektromagnetischen Strahlung, mit dem die zu behandelnden Körperoberflächen des Patienten bestrahlt werden können.

Die erfindungsgemäße Vorrichtung zeichnet sich dadurch aus, dass die Druckerzeugungseinheit der Schröpftherapieanordnung zur Erzeugung von Vakuumimpulsen mit variabel einstellbarer Amplitude ausgestaltet ist. Die Ventileinheit 12 ermöglicht eine stufenlose Variation der erzeugten Vakuumamplitude und es ist ein **elektronischer Regler 13** zur Regelung der erzeugten Vakuumamplitude durch Ansteuerung der Druckerzeugungseinheit und/oder der Ventileinheit 12 vorhanden. Die halboffenen Schröpfgefäße 1 sind aus flexiblem Material aufgebaut und auf einer Vorderseite einer flexiblen Kunststoffmatte angeordnet, wobei die Schröpfgefäße 1 jeweils über eine **Durchgangsöffnung 4** mit der gegenüberliegenden **Rückseite 5** der flexiblen Kunststoff matte verbunden sind. Außerdem ist eine **Deckeleinheit 2** vorgesehen, die im Betriebszustand zusammen mit der Rückseite 5 der flexiblen Kunststoffmatte einen Hohlraum einschließt, in welchen eine **Druckluftleitung 6** mündet, die mit der Druckerzeugungseinheit verbunden ist. Die Lichtquelle 3 der Farblichttherapieanordnung ist in dem von der flexiblen Kunststoffmatte und der Deckeleinheit 2 eingeschlossenen Hohlraum angeordnet, und es ist eine elektronische Steuereinrichtung vorhanden, mit der die Schröpftherapieanordnung und die Farblichttherapieanordnung derart angesteuert werden können, dass sie entweder in Kombination miteinander simultan oder auch jeweils einzeln für sich betreibbar sind.

Die Druckerzeugungseinheit kann Vakuumimpulse mit variabel einstellbarer Frequenz erzeugen. Ein elektronischer Regler ist vorhanden, der zwei Regeleinheiten umfasst, von denen die erste ein von der Druckerzeugungseinheit erzeugtes Grundvakuum, die zweite dem Grundvakuum überlagerte Vakuumimpulse bezüglich ihrer Amplitude, vorzugsweise auch bezüglich ihrer Frequenz, regelt.

Die Lichtquelle 3 der Farblichttherapieanordnung kann farbiges Licht von wählbarer, unterschiedlicher Wellenlänge und wählbarer, variabler Intensität erzeugen, wobei eine Regelvorrichtung vorgesehen ist, mittels derer sowohl die Energie als auch die Amplitude des von der Lichtquelle 3 erzeugten Farblichts geregelt werden kann. Diese Regelvorrichtung ist in der Regel in dem von der flexiblen Kunststoffmatte und der Deckeleinheit 2 umschlossenen Hohlraum angeordnet.

In der in Fig. 1 gezeigten Ausführungsform umfasst die Lichtquelle 3 der Farblichttherapieanordnung eine Vielzahl von Beleuchtungselementen, insbesondere **Lichtleiter 7** und/oder farbige LEDs und/oder OLEDs, welche auf der Vorderseite oder mit optischer Verbindung zur Vorderseite der flexiblen Kunststoffmatte geometrisch auf Lücke zu den Schröpfgefäßen 1 angeordnet sind.

Bei in der Zeichnung nicht eigens dargestellten Ausführungsformen der Erfindung kann die Lichtquelle 3 aber auch flächig ausgedehnt gestaltet sein.

Sämtliche im Hohlraum zwischen der flexiblen Kunststoffmatte und der Deckeleinheit 2 angeordneten optischen und elektronischen Bauteile sind jeweils fluiddicht gekapselt und vorzugsweise auch mechanisch flexibel gestaltet.

Außerdem kann -wie in Fig. 2 dargestellt- eine **Desinfektionsbadewanne 9** vorgesehen sein, in welcher die Baueinheit aus flexibler Kunststoffmatte und Deckeleinheit 2 einschließlich in deren Hohlraum angeordneter Bauteile mittels Desinfektionsflüssigkeit desinfiziert werden kann. In die Desinfektionsbadewanne 9 ist eine elektrische **Ladestation 8** zur berührungslosen Aufladung von **elektrisch aufladbaren Bauteilen 10** integriert, welche in dem von der flexiblen Kunststoffmatte und der Deckeleinheit 2 umschlossenen Hohlraum als autarke Energiequelle zur Versorgung der Lichtquelle 3 angeordnet sind. Die Energiequelle kann Batterien und/oder wiederaufladbare Akkumulatoren und/oder Supercap-Kondensatoren enthalten. Die Ladestation 8 ist an ein im Bereich der Medizintechnik zugelassenes **Netzteil 14** angeschlossen, welches nur einen maximalen Ableitstrom I < 10 µA abgibt. Der Netzanschluss des Netzteils 14 wird üblicherweise folgende elektrische Kenngrößen aufweisen:
90-260 VAC, 50-60 Hz.

## Patentansprüche

1. Vorrichtung zur Ermöglichung einer simultanen Durchführung einer Schröpftherapie und einer Farblichttherapie, wobei die Vorrichtung sowohl eine Schröpftherapieanordnung mit einer Druckerzeugungseinheit zur Erzeugung von Vakuum in einem oder mehreren halboffenen Schröpfgefäßen (1), die mit ihren offenen Seiten zu behandelnde Körperoberflächen eines Patienten abdecken, einer Pumpe (11) zur Vakuumerzeugung sowie einer Ventileinheit (12) als auch eine Farblichttherapieanordnung mit einer Lichtquelle (3) zur Erzeugung von Licht in einem Frequenzbereich innerhalb des sichtbaren Spektrums der elektromagnetischen Strahlung umfasst, mit dem die zu behandelnden Körperoberflächen des Patienten bestrahlt werden können, wobei die Druckerzeugungseinheit der Schröpftherapieanordnung zur Erzeugung von Vakuumimpulsen mit variabel einstellbarer Amplitude ausgestaltet ist, wobei die Ventileinheit (12) eine stufenlose Variation der erzeugten Vakuumamplitude ermöglicht, und wobei ein elektronischer Regler (13) zur Regelung der erzeugten Vakuumamplitude durch Ansteuerung der Druckerzeugungseinheit und/oder der Ventileinheit (12) vorhanden ist,
dass die halboffenen Schröpfgefäße (1) aus flexiblem Material aufgebaut und auf einer Vorderseite einer flexiblen Kunststoffmatte angeordnet sind, wobei die Schröpfgefäße (1) jeweils über eine Durchgangsöffnung (4) mit der gegenüberliegenden Rückseite (5) der flexiblen Kunststoffmatte verbunden sind,
dass eine Deckeleinheit (2) vorgesehen ist, die im Betriebszustand zusammen mit der Rückseite (5) der flexiblen Kunststoffmatte einen Hohlraum einschließt, in welchen eine Druckluftleitung (6) mündet, die mit der Druckerzeugungseinheit verbunden ist,
dass die Lichtquelle (3) der Farblichttherapieanordnung in dem von der flexiblen Kunststoffmatte und der Deckeleinheit (2) eingeschlossenen Hohlraum angeordnet ist,
und dass eine elektronische Steuereinrichtung vorhanden ist, mit der die Schröpftherapieanordnung und die Farblichttherapieanordnung derart angesteuert werden können, dass sie entweder in Kombination miteinander simultan oder auch jeweils einzeln für sich betreibbar sind.

2. Vorrichtung nach Anspruch 1, **dadurch gekennzeichnet, dass** die halboffenen Schröpfgefäße (1) sowie die flexible Kunststoffmatte, vorzugsweise auch die Deckeleinheit (2), aus demselben flexiblen Material, insbesondere aus thermoplastischen Elastomeren, Silikon oder Polypropylen aufgebaut sind.

3. Vorrichtung nach Anspruch 1 oder 2, **dadurch gekennzeichnet, dass** die halboffenen Schröpfgefäße (1) einstückig mit der flexiblen Kunststoffmatte ausgeführt sind.

4. Vorrichtung nach einem der vorhergehenden Ansprüche, **dadurch gekennzeichnet, dass** die Druckerzeugungseinheit Vakuumimpulse mit variabel einstellbarer Frequenz erzeugen kann.

5. Vorrichtung nach einem der vorhergehenden Ansprüche, **dadurch gekennzeichnet, dass** ein elektronischer Regler vorhanden ist, der zwei Regeleinheiten umfasst, von denen die erste ein von der Druckerzeugungseinheit erzeugtes Grundvakuum, die zweite dem Grundvakuum überlagerte Vakuumimpulse bezüglich ihrer Amplitude, vorzugsweise auch bezüglich ihrer Frequenz, regelt.

6. Vorrichtung nach einem der Ansprüche 1 bis 5, **dadurch gekennzeichnet, dass** die flexible Kunststoffmatte aus einem transparenten oder opaken Material aufgebaut ist, wobei die dem Hohlraum zugewandte Seite der Deckeleinheit (2) vorzugsweise verspiegelt ist.

7. Vorrichtung nach einem der Ansprüche 1 bis 5, **dadurch gekennzeichnet, dass** die Lichtquelle der Farblichttherapieanordnung eine Vielzahl von Beleuchtungselementen, insbesondere Lichtleiter (7) und/oder farbige LEDs und/oder OLEDs, umfasst, welche auf der Vorderseite oder mit optischer Verbindung zur Vorderseite der flexiblen Kunststoffmatte geometrisch auf Lücke zu den Schröpfgefäßen (1) angeordnet sind.

8. Vorrichtung nach einem der vorhergehenden Ansprüche, **dadurch gekennzeichnet, dass** sämtliche im Hohlraum zwischen der flexiblen Kunststoffmatte und der Deckeleinheit (2) angeordneten optischen und elektronischen Bauteile jeweils fluiddicht und vorzugsweise auch elastisch gekapselt sind.

9. Vorrichtung nach Anspruch 8, **dadurch gekennzeichnet, dass** eine Desinfektionsbadewanne (9) vorgesehen ist, in welcher die Baueinheit aus flexibler Kunststoffmatte und Deckeleinheit (2) einschließlich in deren Hohlraum angeordneter Bauteile mittels Desinfektionsflüssigkeit desinfiziert werden kann, und dass in die Desinfektionsbadewanne (9) eine elektrische Ladestation (8) zur Aufladung von elektrisch aufladbaren Bauteilen (10) im Hohlraum integriert ist.

10. Vorrichtung nach einem der vorhergehenden Ansprüche, **dadurch gekennzeichnet, dass** die Lichtquelle (3) der Farblichttherapieanordnung farbiges Licht von wählbarer, unterschiedlicher Wellenlänge und wählbarer, variabler Intensität erzeugen kann, und dass eine Regelvorrichtung vorgesehen ist, mittels derer sowohl die Energie als auch die Amplitude des von der Lichtquelle (3) erzeugten Farblichts geregelt werden kann.

11. Vorrichtung nach Anspruch 10, **dadurch gekennzeichnet, dass** die Regelvorrichtung für das von der Lichtquelle (3) erzeugte Farblicht in dem von der flexiblen Kunststoffmatte und der Deckeleinheit (2) umschlossenen Hohlraum angeordnet ist.

12. Vorrichtung nach einem der vorhergehenden Ansprüche, **dadurch gekennzeichnet, dass** in dem von der flexiblen Kunststoffmatte und der Deckeleinheit (2) umschlossenen Hohlraum eine autarke Energiequelle zur Versorgung der Lichtquelle (3) angeordnet ist, wobei die Energiequelle vorzugsweise Batterien und/oder wiederaufladbare Akkumulatoren und/oder Supercap-Kondensatoren enthält.

13. Vorrichtung nach einem der vorhergehenden Ansprüche, **dadurch gekennzeichnet, dass** zumindest die in dem von der flexiblen Kunststoffmatte und der Deckeleinheit (2) umschlossenen Hohlraum angeordneten Teile der Farblichttherapieanordnung mechanisch flexibel gestaltet sind.

14. Vorrichtung nach einem der Ansprüche 1 bis 13, **dadurch gekennzeichnet, dass** die Lichtquelle (3) der Farblichttherapieanordnung eine Vielzahl von quasi-punktförmigen Farblichterzeugenden Elementen (7), vorzugsweise LEDs und/oder OLEDs, umfasst.

15. Vorrichtung nach einem der Ansprüche 1 bis 13, **dadurch gekennzeichnet, dass** die Lichtquelle (3) der Farblichttherapieanordnung flächig ausgedehnt gestaltet ist.

## Claims

1. Device for making it possible to carry out cupping therapy and colour light therapy simultaneously, in which the device comprises both a cupping therapy arrangement with a pressure generating unit for producing a vacuum in one or several half-open cupping vessels (1), which cover the surfaces of the body of a patient to be treated with their open sides, a pump (11) for producing a vacuum and a valve unit (12) as well as a colour light therapy arrangement with a light source (3) for producing light in a frequency range within the visible spectrum of electromagnetic radiation, with which the surfaces of the body of the patient to be treated may be irradiated,
in which
the pressure generating unit of the cupping therapy arrangement is made to produce vacuum impulses with a variably adjustable amplitude, in which the valve unit (12) makes it possible to vary the amplitude of the vacuum produced continuously and in which there is an electronic regulator (13) for regulating the amplitude of the vacuum produced by controlling the pressure generating unit and/or the valve unit (12),
the half-open cupping vessels (1) are made of a flexible material and are arranged on a front of a flexible plastic mat, in which the cupping vessels (1) are connected to the back (5) of the flexible plastic mat lying opposite through an opening (4),
a cover unit (2) is provided, which in the operating state together with the back (5) of the flexible plastic mat encloses a cavity, into which a compressed air line (6) opens, which is connected to the pressure generating unit,
the light source (3) of the colour light therapy arrangement is arranged in the cavity enclosed by the flexible plastic mat and the cover unit (2) and there is an electronic control device, with which the cupping therapy arrangement and the colour light therapy arrangement may be controlled in such a way that they may be operated either simultaneously in combination with each other or also individually by themselves.

2. Device according to claim 1, **characterised in that** the half-open cupping vessels (1) as well as the flexible plastic mat, preferably also the cover unit (2), are made from the same flexible material, particularly from thermoplastic elastomers, silicon or polypropylene.

3. Device according to claim 1 or 2, **characterised in that** the half-open cupping vessels (1) are made in one piece with the flexible plastic mat.

4. Device according to one of the previous claims, **characterised in that** the pressure generating unit may produce vacuum impulses with a variably adjustable frequency.

5. Device according to one of the previous claims, **characterised in that** there is an electronic regulator, which comprises two regulating units, the first of which regulates a basic vacuum produced by the pressure generating unit and the second regulates vacuum impulses overlaying the basic vacuum with reference to their amplitude, preferably also with reference to their frequency.

6. Device according to one of claims 1 to 5, **characterised in that** the flexible plastic mat is made of a transparent or opaque material, in which the side of the cover unit (2) facing the cavity preferably is covered with mirrors.

7. Device according to one of claims 1 to 5, **characterised in that** the light source of the colour light therapy arrangement comprises a large number of lighting elements, particularly light guides (7) and/or colour LEDs and/or OLEDs, which are arranged on the front or with an optical connection to the front of the flexible plastic mat geometrically to the cupping vessels (1) in the gap.

8. Device according to one of the previous claims, **characterised in that** all the optical and electronic components arranged in the cavity between the flexible plastic mat and the cover unit (2) are encased in a fluid-tight way and preferably also elastically.

9. Device according to claim 8, **characterised in that** a disinfecting bath (9) is provided, in which the assembly made of the flexible plastic mat and cover unit (2), including components arranged in its cavity, may be disinfected by means of a disinfecting liquid and an electrical charging station (8) for charging electrically chargeable components (10) in the cavity is integrated into the disinfecting bath (9).

10. Device according to one of the previous claims, **characterised in that** the light source (3) of the colour light therapy arrangement may produce coloured light with a different wavelength selectable and a different intensity selectable and a regulating unit is provided, by means of which both the energy as well as the amplitude of the coloured light produced by the light source (3) may be regulated.

11. Device according to claim 10, **characterised in that** the regulating device for the coloured light produced by the light source (3) is arranged in the cavity enclosed by the flexible plastic mat and the cover unit (2).

12. Device according to one of the previous claims, **characterised in that** a self-sufficient source of energy for supplying the light source (3) is arranged in the cavity enclosed by the flexible plastic mat and the cover unit (2), in which the source of energy preferably contains batteries and/or rechargeable accumulators and/or super capacitors.

13. Device according to one of the previous claims, **characterised in that** at least the parts of the colour light therapy arrangement arranged in the cavity enclosed by the flexible plastic mat and the cover unit (2) are made mechanically flexible.

14. Device according to one of claims 1 to 13, **characterised in that** the light source (3) of the colour light therapy arrangement comprises a large number of almost dot-shaped elements producing coloured light (7), preferably LEDs and/or OLEDs.

15. Device according to one of claims 1 to 13, **characterised in that** the light source (3) of the colour light therapy arrangement is made extensively flat.

## Revendications

1. Dispositif permettant une réalisation simultanée d'une thérapie par ventouses et d'une chromothérapie, dans lequel le dispositif comprend à la fois un dispositif de thérapie par ventouses comportant une unité de génération de pression pour générer un vide dans une ou plusieurs coupe(s) de ventouse semi-ouverte(s) (1) qui couvrent de leurs côtés ouverts des surfaces corporelles à traiter d'un patient, une pompe (11) pour générer un vide ainsi qu'une unité de soupape (12) et également un dispositif de chromothérapie comportant une source de lumière (3) pour générer de la lumière dans une plage de fréquence au sein du spectre visible du rayonnement électromagnétique, avec laquelle les surfaces corporelles à traiter du patient peuvent être irradiées,
dans lequel l'unité de génération de pression du dispositif de thérapie par ventouses est conçu pour générer des impulsions de vide avec des amplitudes ajustables de façon variable, dans lequel l'unité de soupape (12) permet une variation douce de l'amplitude de vide généré, et dans lequel un système de réglage électronique (13) est présent pour le réglage de l'amplitude de vide généré par une commande de l'unité de génération de pression et/ou de l'unité de soupape (12),
les coupes de ventouse semi-ouvertes (1) sont constituées de matériau flexible et sont disposées sur un côté avant d'un tapis flexible en matière plastique, dans lequel les coupes de ventouse (1) sont reliées respectivement par une ouverture traversante (4) avec le côté arrière opposé (5) du tapis flexible en matière plastique,
une unité de couvercle (2) est prévue, qui renferme à l'état de fonctionnement conjointement avec le côté arrière (5) du tapis flexible en matière plastique, une cavité dans laquelle débouche une conduite d'air comprimé (6) qui est reliée avec l'unité de génération de pression,
la source de lumière (3) du dispositif de chromothérapie est disposée dans la cavité renfermée par le tapis en matière plastique et l'unité de couvercle (2),
et un dispositif de commande électronique est présent, avec lequel le dispositif de thérapie par ventouses et le dispositif de chromothérapie peuvent être commandés de telle sorte qu'ils peuvent être actionnés soit simultanément de façon combinée l'un avec l'autre, soit également, respectivement individuellement chacun pour soi.

2. Dispositif selon la revendication 1, **caractérisé en ce que** les coupes de ventouses semi-ouvertes (1) ainsi que le tapis en matière plastique flexible, de préférence également l'unité de couvercle (2), sont constitués du même matériau flexible, en particulier d'élastomères thermoplastiques, de silicone ou de polypropylène.

3. Dispositif selon la revendication 1 ou 2, **caractérisé en ce que** les coupes de ventouse semi-ouvertes (1) sont réalisées d'un seul tenant avec le tapis en matière plastique flexible.

4. Dispositif selon l'une quelconque des revendications précédentes, **caractérisé en ce que** l'unité de génération de pression peut générer des impulsions de vide avec une fréquence pouvant être ajustée de façon variable.

5. Dispositif selon l'une quelconque des revendications précédentes, **caractérisé en ce qu'**un système de réglage électronique est présent, qui comprend deux unités de réglage, parmi lesquelles la première règle un vide de base généré par l'unité de génération de pression, la deuxième règle des impulsions de vide superposées au vide de base concernant leur amplitude, de préférence également concernant leur fréquence.

6. Dispositif selon l'une quelconque des revendications 1 à 5, **caractérisé en ce que** le tapis en matière plastique flexible est constitué d'un matériau transparent ou opaque, dans lequel le côté de l'unité de couvercle (2) tourné vers la cavité est de préférence réfléchissant.

7. Dispositif selon l'une quelconque des revendications 1 à 5, **caractérisé en ce que** la source de lumière du dispositif de chromothérapie comprend une pluralité d'éléments d'éclairage, en particulier un conduit de lumière (7) et/ou des LED de couleur et/ou des OLED, qui sont disposées géométriquement sur le côté avant ou en liaison optique avec le côté avant du tapis en matière plastique flexible sur des intervalles par rapport aux coupes de ventouse (1).

8. Dispositif selon l'une quelconque des revendications précédentes, **caractérisé en ce que** tous les composants optiques et électroniques disposés dans la cavité entre le tapis en matière plastique flexible et l'unité de couvercle (2) sont respectivement étanches aux fluides et de préférence également encapsulés élastiquement.

9. Dispositif selon la revendication 8, **caractérisé en ce qu'**un bain de désinfection (9) est prévu, dans lequel l'unité modulaire constituée d'un tapis en matière plastique flexible et d'une unité de couvercle (2) y compris des éléments disposés dans leur cavité peut être désinfectée au moyen de liquide de désinfection, et **en ce que** dans le bain de désinfection (9), un poste de charge électrique (8) est intégré dans la cavité pour le chargement d'éléments pouvant être chargés électriquement (10).

10. Dispositif selon l'une des revendications précédentes, **caractérisé en ce que** la source de lumière (3) du dispositif de chromothérapie peut générer une lumière colorée de longueurs d'ondes différentes sélectionnables et d'intensité variable sélectionnable, et **en ce qu'**un dispositif de réglage est prévu, au moyen duquel aussi bien l'énergie qu'également l'amplitude de la lumière colorée générée par la source de lumière (3) peut être réglée.

11. Dispositif selon la revendication 10, **caractérisé en ce que** le dispositif de réglage pour la lumière colorée générée par la source de lumière (3) est disposé dans la cavité entourée par le tapis en matière plastique flexible et l'unité de couvercle (2).

12. Dispositif selon l'une des revendications précédentes, **caractérisé en ce que** dans la cavité entourée par le tapis en matière plastique flexible et l'unité de couvercle (2) est disposée une source d'énergie autonome pour alimenter la source de lumière (3), dans lequel la source d'énergie contient de préférence des batteries et/ou des accumulateurs rechargeables et/ou des supercondensateurs.

13. Dispositif selon l'une des revendications précédentes, **caractérisé en ce qu'**au moins les pièces disposées dans la cavité entourée par le tapis en matière plastique flexible et l'unité de couvercle (2) du dispositif de chromothérapie sont de configuration mécanique flexible.

14. Dispositif selon l'une quelconque des revendications 1 à 13, **caractérisé en ce que** la source de lumière (3) du dispositif de chromothérapie comprend une multiplicité d'éléments générant une lumière colorée (7) quasiment sous forme de points, de préférence des LED et/ou des OLED.

15. Dispositif selon l'une quelconque des revendications 1 à 13, **caractérisé en ce que** la source de lumière (3) du dispositif de chromothérapie est de configuration plane étendue.
